# EUROPÄISCHE PATENTANMELDUNG

(11) **EP 1 466 593 A1**
(43) Veröffentlichungstag der Anmeldung: **13.10.2004**
(21) Anmeldenummer: 04007530.1
(22) Anmeldetag: 29.03.2004
(51) Int. Cl.: A61K 9/00, A61L 27/18, A61L 27/54

(54) **Biodegradierbares injizierbares Implantat**

(30) Priorität: 28.03.2003 DE 10314082
(71) Anmelder: MCS Micro Carrier Systems GmbH, 41460 Neuss (DE)
(72) Erfinder: Schreier, Hans Dr., 41464 Neuss (DE); Souad Benahmed, Amina, 41460 Neuss (DE)
(74) Vertreter: Christophersen & Partner

(57) **Zusammenfassung**

Ein biodegradierbares Implantat enthaltend (a) ein Co-polymer Poly(Milchsäure-co-Glycolsäure) (PLGA), (b) ein biokompatiblen Lösungsmittel, mischbar mit Wasser oder Körperflüssigkeiten, und (c) einen Arzneistoff, eine Inhibitorsubstanz, und/oder eine andere biologisch aktive Substanz.

## Beschreibung

Die vorliegende Erfindung betrifft ein injizierbares biodegradierbares Implantat als Medizinprodukt zur Anwendung im tierischen und menschlichen Körper.

### Hintergrund

Die Anwendung von Biopolymeren, z.B. als Wundverschluss, chirurgische Klammern, Vorrichtungen zur Freisetzung von Arzneistoffen, und rekonstruktive Implantate (Wu, in 'Encydopedic Handbook of Biomaterials and Bioengineering (Wise et al., eds.), Marcel Dekker, New York, NY, 1995, 1015-1054; Chegini et al., J. Reprod. Med., 33 (1988) 187-192; Menei et al., Neurosurgery, 4, (1994) 1058-1064) ist bekannt. Die Mehrzahl dieser biologisch abbaubaren Polymere bestehen aus thermoplastischen Materialien auf der Basis von Glykoliden, Laktiden, ε-Caprolaktonen und deren Co-polymeren.

Systeme zur Freisetzung von Arzneistoffen, die diese Polymere einsetzen, werden normalerweise außerhalb des Körpers hergestellt, wobei der Arzneistoff in das Polymer eingearbeitet wird und die Mischung dann in eine bestimmte Form wie ein Zylinder, Paste, Scheibe, oder Fäden für die Implantation gebracht wird. Das System wird dann durch einen chirurgischen Einschnitt in den Körper eingebracht. Diese Systeme können auch als Mikrosphären oder Mikrokapseln für die Injektion mit einer Spritze formuliert werden, um ein chirurgisches Implantat zu vermeiden. Mikrosphären und Mikrokapseln erfordern normalerweise den Einsatz von Lösungsmitteln oder hoher Temperatur, was die Stabilität des Arzneistoffs negativ beeinflussen kann; auch müssen sie vor der Injektion rekonstituiert werden. Nach erfolgter Injektion ist die Deposition von Mikrosphären und Mikrokapseln ohne den Einsatz extensiver chirurgischer Maßnahmen irreversibel.

Die in situ Formulierung biodegradierbarer Implantate ist z.B. in den US Patenten Nrn. 4,938,763; 6,206,920; and 6,461,631, sowie bei Lambert et al., J. Controlled Release 33 (1995) 189-195, Shah et al., J. Controlled Release 27 (1993) 139-147, und Chandrashekar et al., J. Pharm. Pharmacol. 48 (1996), 669-674, beschrieben.

Im US Patent No. 4,938,763 beschreiben Dunn et al. ein alternatives in situ Implantat, indem sie ein nicht-reaktives, wasser-unlösliches thermoplastisches Polymer in einem biokompatiblen, mit Wasser mischbaren Lösungsmittel unter Bildung einer Flüssigkeit lösen, die resultierende Lösung in den Körper einbringen, und unter Entfernen des Lösungsmittel ein festes Implant herstellen. Nach intramuskulärer oder subkutaner Injektion diffundiert das wasserlösliche Lösungsmittel aus dem Polymer, während Wasser in die Polymermatrix eindiffundiert. Auf Grund seiner Wasserunlöslichkeit koaguliert und verfestigt es sich schnell in eine feste Matrixstruktur. Wenn ein solches Implantat als Arzneistoffträger verwendet wird, wird der Arzneistoff in die Lösung vor Injektion eingebracht und wird dann in der festen Matrix nach erfolgter Koagulierung des Polymers festgehalten. Unterschiedliche Koagulationsgrade und damit der Unterschiedliche Grade der Biodegradierbarkeit und der Arzneistofffreisetzung, können erhalten werden durch variieren der Charakteristika der Polymere und Co-polymere, des Lösungsmittels, und der Mengenverhältnisse der einzelnen Komponenten (einschließlich des Arzneistoffs) zueinander vor der Injektion.

Dunn et al. beschreiben verschiedene Methoden zur Herstellung von Lösungsmittel-Polymer- Kombinationen zur Verwendung als in situ biodegradierbare Implantate, von denen viele den Einsatz von Katalysatoren, Lösungsmittelsystemen, erhöhte Temperatur, und andere Bedingungen zur Verabreichung solcher Lösungsmittel-Polymer-Lösungen erfordern.

Shah et al., und auch Eliaz et al. im US Patent 6,206,920 schlagen neue Materialien für die Herstellung biodegradierbarer Implantate vor, die für die Anwendung im Menschen erlaubt sind. Sie schlagen ein System vor, das aus dem wasser-unlöslichen Co-polymer Poly(Milchsäure-co-Glycolsäure) (PLGA) besteht, das in einem pharmazeutisch akzeptierbaren wasser-mischbaren Lösungsmittel gelöst ist.

Shah et al. benutzen Triacetin, das als Plastizicer und Lebensmittelzusatz bekannt ist, während Eliaz et al. Glycofurol (α-Tetrahydrofuranyl-θ-hydroxypoly(oxy-1,2-ethandiyl) benutzen, das als parenterales Lösungsmittel für die intravenöse oder intramuskuläre Injektionen in Konzentrationen bis zu 50% v/v eingesetzt wird. Diese Lösungsmittel wurden zur Herstellung injizierbarer Lösungen verschiedener Arzneistoffe verwendet wie z.B. für Dinoproston (Kimball, Prostaglandins, 32 (1986) 527-537), Diazepam (Loscher & Honack, Epilepsia, 38 (1997) 106-113), Antibiotika (Shultz-Coulon, Fortschr. Med., 26 (1982) 1268-1272), und Hormone (Taubolland et al., Epilepsy Res., 7 (1990) 59-64).

Bezüglich Nervengewebe wurden aliphatische Polyester als kompatible Substanzen im peripheren Nervensystem (de Medinaceli et al., J. Reconstr. Microsurg., 11 (1995), 43-49), im Zentralnervensystem (ZNS) (Kou et al., J. Controlled Release, 43 (1997) 123-130), und im Rückenmark (Gautier et al., J. Biomed. Mater. Res., 42 (1998), 642-54; Friedman et al., Neurosurgery 51 (2002) 742-751) eingesetzt. Zur Reparatur durchtrennter peripherer Nerven, wurden tubuläre Führungskanäle aus verschiedenen Polymerzusammensetzungen hergestellt und im Tierversuch (Oudega et al., Biomaterials, 22 (2001) 1125-1136; Hadlock et al. Tissue Eng., 6 (2000) 119-127) bzw. im Humanversuch (MacKinnon & Dellon, Plast. Reconstr. Surg. 85 (1990) 419-424) getestet. Diese Polyester und deren Abbauprodukte erzeugten in diesen Studien keine entzündlichen Prozesse über das normale, in verletztem peripherem Nervengewebe beobachtete Maß hinaus.

Ein Haupthindernis für die axonale Regenerierung nach einem Trauma des erwachsenen Zentralnervensystems ist die Produktion spezifischer extrazellulärer Matrixmoleküle. Insbesondere kann die Deponierung von Collagen III zusammen mit der Basalmembran (BM) (Collagen IV und Lamina) mit dem Verlust von Axonen im ZNS auch über den eigentlichen Bereich der Verletzung hinaus beobachtet werden (WO 98/5170; Weidner et al., Eur. J. Neurosci. 160 (1999) 40-50).

In der WO 98/51708 beschreiben Müller et al. eine Methode zur Verbesserung verletzten neuronalen Gewebes durch die lokale Injektion von polyklonalen Anti-collagen IV-Antikörpern oder des Eisenkomplexbildners (2,2')-Dipyridyl. Es konnte gezeigt werden, dass Dipyridyl die Triple-Helix- Ausbildung von Collagen verhindert, während nach der Injektion von Anti-Collagen IV und Dipyridyl die Ablagerung einer Basalmembrane (BM) an der Läsionsstelle merkbar reduziert war.

Weidner et al. zeigten ähnliche Ergbinsse bei der Injektion des Eisenkomplexbildners 2,2'-Bipyridin. Diese Ergebnisse und die Reduktion von Collagen und BM deuten darauf hin, dass Collagen und die Ausbildung einer Basalmembran nach einer Verletzung des ZNS das Wachstum von Axonen beeinträchtigen, und deshalb zusammen blockiert werden müssen, wenn die axonale Regeneration angeregt werden soll.

Es ist deshalb Aufgabe dieser Erfindung, ein einfaches System bereitzustellen, das mit der Biosynthese von Collagen interferiert, und eine Methode zu seiner Herstellung.

Eine weitere Aufgabe dieser Erfindung ist, ein flüssiges System bereitzustellen, das einfach zusammen mit einem Inhibitor wie z.B. einem Eisenchelator formuliert und verabreicht werden kann.

Eine weitere Aufgabe dieser Erfindung ist, ein flüssiges System, das als Reservoir für einen Arzneistoff oder eine Inhibitorsubstanz wie z.B. einen Eisenchelator dient, bereitzustellen.

Es ist eine weitere Aufgabe dieser Erfindung, eine Methode zur kontinuierlichen Aufnahme von Eisenionen und deren Bindung an eine Inhbitorsubstanz in einem einfachen flüssigen System bereitzustellen.

Gegenstand dieser Erfindung ist, ein biodegradierbares Implantat enthaltend (a) ein Copolymer Poly(Milchsäure-co-Glycolsäure) (PLGA), (b) ein biokompatibles Lösungsmittel, das mit Wasser oder Körperflüssigkeiten mischbar ist, und (c) ein Arzneistoff, eine Inhibitorsubstanz, und/oder eine andere biologisch aktive Substanz.

Ein weiterer Gegenstand dieser Erfindung ist eine Methode zur in situ Herstellung des biodegradierbaren Implantats bereitzustellen, das die folgenden Schritten umfasst:
a. Auflösen von Poly(Milchsäure-co-Glycolsäure) (PLGA) in einem Lösungsmittel, das entweder Triacetin oder Glycofurol sein kann, unter Erhitzen, um eine Lösung herzustellen;
b. Administration der notwendigen Menge dieser Lösung in den tierischen oder menschlichen Körper;
c. Verteilung des Lösungsmittels im tierischen oder menschlichen Körper, während sich ein festes Implantat aus der PLGA Polymermatrix bildet;
d. Lösung, Dispersion, oder Suspension eines Arzneistoffs, einer Inhibitorsubstanz oder eines anderen biologisch aktiven Moleküls in der Lösung nach Dissolution des Polymers;
e. Komplexierung des Arzneistoffs, der Inhibitorsubstanz, und/oder eines anderen biologisch aktiven Moleküls mit Eisenionen in besagtem PLGA Polymersystem.

Angewandt als flüssiges System, besteht das Implantat aus einem biodegradierbaren, wasser-unlöslichen thermoplastischen Polymer und einem wasser-mischbaren Lösungsmittel. Das flüssige System wird fest in situ nach Injektion an der Stelle des gewünschten Implantats, wo es eine mikroporöse Matrix ausbildet, nachdem sich das Lösungsmittel in der umliegenden Gewebeflüssigkeit verteilt und das Polymer koaguliert. Das System stellt eine einspritzbare, feste biodegradierbare Arzneiform oder ein Reservoirsystem dar, dem eine effektive Menge Arzneistoff, Inhibitorsubstanz, oder andere biologisch aktive Substanz zu der Flüssigkeit vor der Injektion zugegeben wird. Der Arzneistoff, die Inhibitorsubstanz, oder ein anderes biologisch aktives Moleküls ist eingeschlossen in der festen Matrix nach Koagulation des Polymers. Die Zusammensetzung kann im Körper angewandt werden um neurologische Krankheiten wie Rückgradverletzungen oder andere Nervengewebsdefekte zu behandeln, oder um die Nervenregenration anzuregen.

### Zusammenfassung

Die vorliegende Erfindung stellt ein in situ Biopolymer-Implantat als Medizinprodukt dar, das als Reservoirsystem für eine Inibitorsubstanz wie z.B. einen Eisenchelator, dienen kann. Die Bindungskapazität für Eisenionen unterstützt die Interferenz mit der Collagenbiosynthese und unterbindet die Ausbildung von Collagen und einer Basalmembran nach einer Rückgratverletzung.

Die Zusammensetzung der Erfindung enthält ein Polymer, das ein Copolymer Poly(Milchsäure-co-Glycolsäure) (PLGA) mit einem Verhältnis der Glycolsäureeinheiten (GA) zu Milchsäureeinheiten (LA) von 15:85 bis 85:5 ist, insbesondere von 25:75 bis 75:25.

Als Komponente (b) ist erfindungsgemäß ein biokompatibles Lösungsmittel, welches mit Wasser oder Körperflüssigkeiten vermischbar ist, enthalten. In einer bevorzugten Ausführungsform wirkt dieses Lösungsmittel auch als Plasticizer. Besonders geeignete Lösungsmittel sind Triacetin und Glycofurol.

Die weitere erfindungsgemäß enthaltene Komponente ist ein Arzneistoff, eine Inhibitorsubstanz oder eine andere biologisch aktive Substanz. Vorzugsweise wird als dritte Substanz ein Eisenkomplexbildner eingesetzt. Dieser kann eine beliebige für die Komplexierung von Eisenionen physiologisch erträgliche und somit im pharmazeutischen Bereich geeignete Verbindung sein. Geeignete Beispiele für Eisenkomplexbildner sind 3,4'-Bipyridin, 2,2'-Bipyridin, und deren pharmazeutisch gebräuchlichen Salzen oder Desferoxamin, Deferepron, Calcium-Trinatrium-Pentetat, Tiopronin sowie Oxalate und Phytine. Besonders geeignet sind solche Eisenkomplexbildner, die die Bildung einer Basalmembran inhibieren.

Geeignete Beispiele für eine Inhibitorsubstanz, die als Komponente (c) enthalten sein kann, werden vorzugsweise ausgewählt aus der Gruppe Antikörper gegen die Collagen Lamina und die Bausteine der Basalmembran, N-Oxaloglycin, Zink Salze, Pyridinderivate und dessen Ethylester und Ätheramide, 3,4'-Dihydroxybenzoat, Prolin und seine strukturellen und funktionellen Analoge β-Aminopropionitril, Anthracycline, 2,78-Trihydroxyanthrachinone, Fibrostatin-C, Coumalinsäure und ihre pharmazeutisch gebräuchliche Salze, 5-Oxaprolin, β-Lactamantibiotica.

Die Komponente (c) kann auch ein Gemisch aus mehreren Substanzen, d.h. aus Arzneistoffen, Inhibitorsubstanzen und/oder anderen biologisch aktiven Substanzen bestehen. Diese sind vorzugsweise so aufeinander abgestimmt, dass sie sich nicht nachteilig beeinflussen und sich in ihrer Wirkung ergänzen bzw. Innerhalb des Implantats unterschiedliche Aufgaben hinsichtlich ihrer Wirkung erfüllen.

Das erfindungsgemäße Implantat bildet sich in situ im Körper. Die in-situ-Bildung ermöglicht es, dass das Implantat als Lösung verabreicht werden kann, beispielsweise durch Injektion mit einer Spritze.

Das erfindungsgemäße Implantat wird vorzugsweise zur Herstellung eines Medikamentes zur Behandlung von Rückgratverletzungen und anderen peripheren oder zentralen Nervenverletzungen eingesetzt.

Ein weiterer Gegenstand der vorliegenden Erfindung ist demgemäß ein Gemisch aus (a) einem Co-polymer aus Poly(Milchsäure-co-Glycolsäure) (PLGA), (b) einem biokompatiblen Lösungsmittel, welches mischbar ist mit Wasser oder Körperflüssigkeiten, und (c) einem Arzneistoff, einer Inhibitorsubstanz und/oder einer anderen biologisch aktiven Substanz zur Herstellung eines Medikaments zur Behandlung von Rückgratverletzungen und anderen peripheren oder zentralen Nervenverletzungen.

### Beschreibung der Figuren

FIG. 1 zeigt die Eisenbindungskapazität von DFOM im PLGA Implantat.
FIG. 2 zeigt die Eisenbindungskapazität verschiedener Konzentrationen von DFOM im PLGA Implantat.
FIG. 3 zeigt die Kinetik der Bildung des Eisenkomplexes.
FIG. 4 zeigt die Eisenbindungskapazität des Implantats mit verschiedenen Plasticizern.

### Beispiele

### Beispiel 1

### Herstellung von Polymerverbindungen, die Deferoxamin (DFOM) enthalten.

Fünf Proben (Tabelle 1) wurde wie folgt hergestellt: Co-polymer Poly(Milchsäure-co-Glycolsäure) (PLGA) (50/50 LA/GA) wurde im entsprechenden Lösungsmittel, Triacetin (T) oder Glycofurol (G) unter Erhitzen auf ungefähr 60°C mit einem Magnetmischer gelöst. Die PLGA Lösung wurde auf Raumtemperatur gekühlt, und das Deferoxamin und ein hydrophobes Additiv Miglyol 812 (0,5g) darin mit einem Homogenisator dispergiert.

**Tabelle 1**

| Probe No | PLGA (g) | Lösungsmittel T or G (g) | DFOM (g) | Formulierung |
|---|---|---|---|---|
| 1 | 0,5 | 4 (T) | 0,05 | 10% PLGA, 10% DFOM |
| 2 | 1 | 3,35 (T) | 0,025 | 20% PLGA, 2,5% DFOM |
| 3 | 1 | 3,45 (T) | 0,05 | 20% PLGA, 5% DFOM |
| 4 | 1 | 3,4 (T) | 0,1 | 20% PLGA, 10% DFOM |
| 5 | 1 | 3,45(G) | 0,05 | 20% PLGA, 5% DFOM |

### Beispiel 2

### In vitro Bindungsprofile

In vitro Bindungsprofile wurden erzeugt, indem 0,2 ml der PLGA Lösung mit Deferoxamin (DFOM) mit Hilfe einer 1-ml Spritze direkt in 16 ml Phosphatpuffer (PBS, pH 7,4) eingespritzt wurden, so dass sich ein festes Implantat, das DFOM enthält, bildete. Nachdem das Implantat geformt und gewaschen war, wurden 8 ml einer Eisenionenlösung zugegeben (Zeit = 0). Die Proben wurden kontinuierlich bei 37°C geschüttelt. Die Eisenchelatorkapazität von DFOM wurde quantitativ colorimetrisch erfasst mit Hilfe von Feren als Chromogen. Die Absorption wurde gegen Puffer bei 592nm gemessen.

### Beispiel 3

### Bestimmung der Chelator Bindungscharakteristika von DFOM im PLGA Implantat.

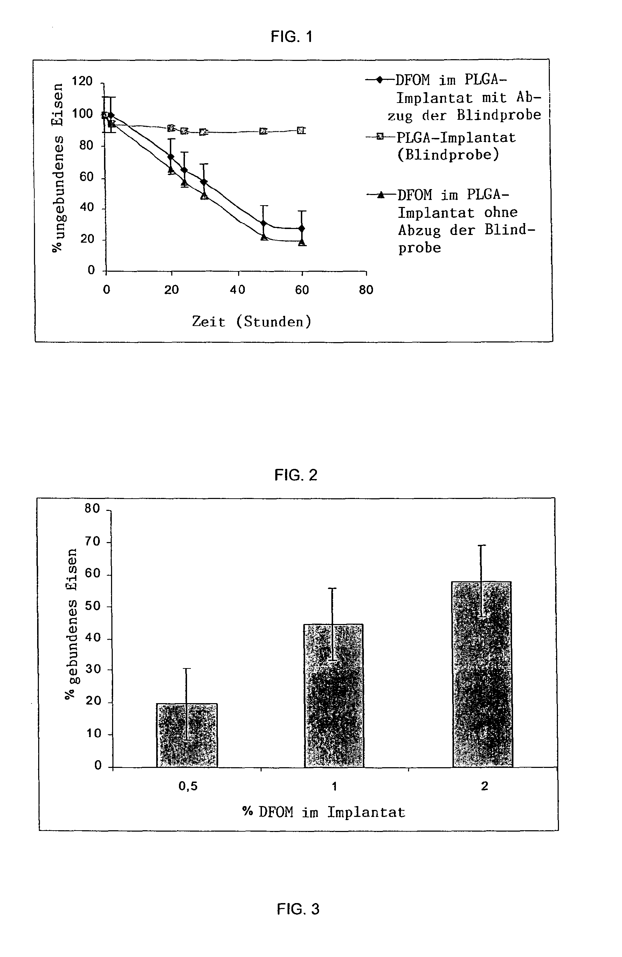

In vitro Bindungsprofile von 3 Proben analog zu den 3 Proben in Tabelle 1 wurden bestimmt. FIG. 1 zeigt die Eisenbindungskapazität von DFOM im PLGA Implantat.

### Beispiel 4

### Kinetik der Eisenbindung mit verschiedenen Konzentration en von DFOM

Zwei Proben analog zu Proben 2, 3, 4 in Tabelle 1 wurden untersucht. FIG. 2 zeigt das Verhältnis die Eisenbindungskapazität von DFOM im PLGA Implantat.

### Beispiel 5

### Kinetik der Eisenkomplexbildung

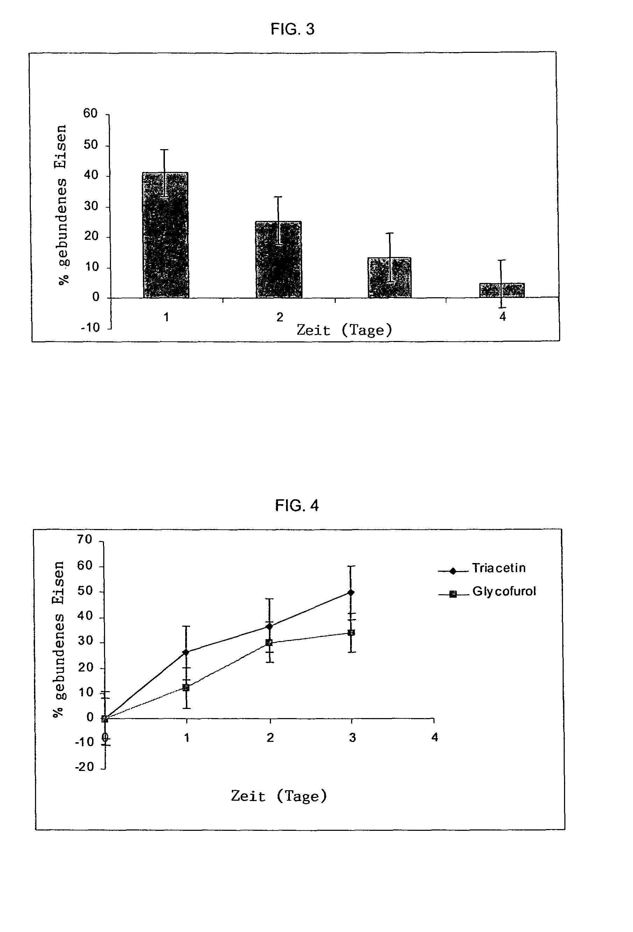

Die Eisenbindungskapazität des Implantat wurde bestimmt, indem eine Probe sequentiell alle 24 Std. in eine frische Eisenionlösung transferiert wurde. Fünf Proben analog zu Probe 3 wurden untersucht. FIG. 3 zeigt, dass die Eisenbindungskapazität des Implantats nach 4 Tagen erschöpft war.

### Beispiel 6

### Kinetik der DFOM-Eisen-Komplexe nach Saturierung.

Saturierte Systeme von Probe 3 (Beispiel 4) wurden in Pufferlösungen (PBS, pH 7,4) transferiert. Das Experiment wurde bei 37°C unter Schütteln durchgeführt. Pufferproben wurden periodisch entnommen und die Absorption des Eisenkomplexes mit Hilfe des Chromogens bei 592 nm gegen Puffer gemessen. Über 4 Wochen konnte kein eisen im Medium nachgewiesen werden.

### Beispiel 7

### Einfluss verschiedener Plasticizer auf die Charakteristika des Implantats.

Zwei Proben analog zu Proben 3 und 5 (Tabelle 1) wurde analog zu Beispiel 3 untersucht. FIG. 4 zeigt das Verhältnis der Plasticizer zur hydrolytischen Spaltung von PLGA und den Einfluss auf die Eisenbindungskapazität.

## Patentansprüche

1. Ein biodegradierbares Implantat enthaltend (a) ein Co-polymer aus Poly(Milchsäure-co-Glycolsäure) (PLGA), (b) ein biokompatibles Lösungsmittel, mischbar mit Wasser oder Körperflüssigkeiten, und (c) einen Arzneistoff, eine Inhibitorsubstanz und/oder eine andere biologisch aktive Substanz.

2. Das biodegradierbare Implantat nach Anspruch 1, wobei das Co-polymer Poly(Milchsäure-co-Glycolsäure) (PLGA) ist.

3. Das biodegradierbare Implantat nach Anspruch 2, **dadurch gekennzeichnet, dass** Copolymer das Verhältnis von Glycolsäureeinheiten (GA) zu Milchsäureeinheiten (LA) von 15:85 bis 85:5, insbesondere von 25:75 bis 75:25 beträgt.

4. Das biodegradierbare Implantat nach Ansprüchen 1 bis 3, wobei das biokompatible Lösungsmittel mit Wasser oder Körperflüssigkeit mischbar und als Plasticizer geeignet ist, wie Triacetin und Glycofurol.

5. Das biodegradierbare Implantat nach Ansprüchen 1 bis 4, wobei der Arzneistoff, die Inhibitorsubstanz oder andere biologisch aktive Substanz ein Eisenkomplexbildner ist.

6. Der Eisenkomplexbildner nach Anspruch 5, bestehend aus 3,4'-Bipyridin, 2,2'-Bipyridin, und deren pharmazeutisch gebräuchlichen Salzen oder Desferoxamin, die als Fe-Komplexbildner die Bildung einer Basalmembran inhibieren.

7. Das biodegradierbare Implantat nach Ansprüchen 1 bis 6, wobei die Inhibitorsubstanz aus der Gruppe wie Antikörper gegen die Collagen Lamina und die Bausteine der Basalmembran, N-Oxaloglycin, Zink Salze, Pyridinderivate und dessen Ethylester und Ätheramide, 3,4'-Dihydroxybenzoat, Prolin und seine strukturellen und funktionellen Analoge β-Aminopropionitril, Anthracycline, 2,7,8-Trihydroxyanthrachinone, Fibrostatin-C, Coumalinsäure und ihre pharmazeutisch gebräuchliche Salze, 5-Oxaprolin, β-Lactam-antibiotica, ist.

8. Eine Methode zur Herstellung eines festen Implantats in situ im Körper, bestehend aus folgenden Schritten:
a. Auflösen von Poly(Milchsäure-co-Glycolsäure) (PLGA) in einem Lösungsmittel, das entweder Triacetin oder Glycofurol sein kann, unter Erhitzen, um eine Lösung herzustellen;
b. Administration der notwendigen Menge dieser Lösung in den tierischen oder menschlichen Körper;
c. Verteilung des Lösungsmittels im tierischen oder menschlichen Körper, während sich ein festes Implantat aus der PLGA Polymermatrix bildet;
d. Lösung, Dispersion, oder Suspension eines Arzneistoffs, einer Inhibitorsubstanz, oder eines anderen biologisch aktiven Moleküls in der Lösung nach Dissolution des Polymers;
e. Komplexierung des Arzneistoffs, der Inhibitorsubstanz, und/oder des anderen biologisch aktiven Moleküls mit Eisenionen in besagtem PLGA Polymersystem.

9. Das Verfahren nach Anspruch 8, wobei die Flüssigkeit durch Injektion mit einer Spritze verabreicht wird.

10. Ein Gemisch aus (a) einem Co-polymer aus Poly(Milchsäure-co-Glycolsäure) (PLGA), (b) einem biokompatiblen Lösungsmittel, welches mischbar ist mit Wasser oder Körperflüssigkeiten, und (c) einem Arzneistoff, einer Inhibitorsubstanz und/oder einer anderen biologisch aktiven Substanz geeignet zur in-situ-Bildung eines Implantats im tierischen oder menschlichen Körper.

11. Verwendung eines Gemisches nach Anspruch 10 zur Herstellung eines Medikaments zur Behandlung von Rückgratverletzungen und anderen peripheren oder zentralen Nervenverletzungen.

12. Verwendung einer Inhibitorsubstanz, die in der Lage ist, die Bildung einer Basalmembran, die durch eine Läsion von Nervengewebe verursacht wurde, zu verhindern oder zu inhibieren, für die Herstellung eines Medikaments nach Anspruch 8.

13. Ein biodegradierbares Implantat, hergestellt nach der Methode in Anspruch 8.
